# EUROPEAN PATENT APPLICATION

(11) **EP 4 047 080 A1**
(43) Date of publication of application: **24.08.2022**
(21) Application number: 22157310.8
(22) Date of filing: 17.02.2022
(51) Int. Cl.: C12N 1/12

(54) **METHOD FOR MICROALGAL CULTIVATION**

(30) Priority: 23.02.2021 KR 20210024087; 19.01.2022 KR 20220008057
(71) Applicant: Korea University Research and Business Foundation, Seoul 02841 (KR)
(72) Inventor: SIM, Sang Jun, 07331 Seoul (KR); JOUN, Jae Min, 07029 Seoul (KR); HONG, Min Eui, 13644 Seongnam-si (KR)
(74) Representative: Schön, Christoph

(57) **Abstract**

Disclosed is a method for microalgal cultivation. The method includes cultivating a microalgal species in a medium in an autotrophic mode for a predetermined first time period where light is supplied and supplying an organic carbon source to the medium to cultivate the microalgal species in a heterotrophic mode for a predetermined second time period where the supply of light is stopped.

## Description

### BACKGROUND OF THE INVENTION

### 1. Field of the Invention

The present invention relates to a method for microalgal cultivation, and more specifically to a method for continuous microalgal cultivation in separate culture modes, *i.e.* in an autotrophic mode at a time when photosynthesis occurs and in a heterotrophic mode at a time when photosynthesis does not occur.

### 2. Description of the Related Art

The commercialization of microalgal bioprocesses is important for carbon dioxide capture and utilization (CCU) and economic feasibility. Autotrophic modes for microalgal cultivation are highly dependent on the concentration of CO₂ that accounts for 0.04% of the atmosphere. The presence of CO₂ at a high concentration enables sufficient mass transfer for microalgal growth. Thus, flue gas from power plants that can supply concentrated CO₂ and meet the economic requirements for CO₂ supply is used for large-scale cultivation of microalgae.

Strategies and studies have been continuously reported to improve the productivity of biomass for the commercialization of microalgal bioprocesses. Existing autotrophic modes suffer from low photosynthetic efficiency after the light cycle for a predetermined time period despite a continuous supply of light and CO₂, limiting an increase in biomass productivity. To overcome the disadvantages of such autotrophic modes, proposals have been made on mixotrophic and heterotrophic modes that can use more organic carbon sources, contributing to an improvement in biomass productivity. A mixotrophic mode refers to a trophic mode in which autotrophic and heterotrophic modes occur together in a single culture process. In a mixotrophic mode, an autotrophic mode, where carbon is fixed from an inorganic carbon source through photosynthesis and is used as chemical energy, and a heterotrophic mode, where energy required for cellular activity is obtained from an organic carbon source through respiration, occur simultaneously. The mixotrophic mode can achieve high biomass productivity compared to the other culture modes. Nevertheless, the mixotrophic mode faces major challenges such as inhibited absorption of concentrated organic carbon, chlorophyll loss due to reduced photosynthetic efficiency, and high supply cost of the organic carbon source.

### SUMMARY OF THE INVENTION

The present invention has been made in an effort to solve the problems of the prior art and an aspect of the present invention is to provide a method for microalgal cultivation in separate culture modes in different cycles, specifically in an autotrophic mode in the light cycle and in a heterotrophic mode in the dark cycle.

A method for microalgal cultivation according to the present invention includes (a) cultivating a microalgal species in a medium in an autotrophic mode for a predetermined first time period where light is supplied and (b) supplying an organic carbon source to the medium to cultivate the microalgal species in a heterotrophic mode for a predetermined second time period where the supply of light is stopped.

In the method of the present invention, steps (a) and (b) may be repeated sequentially.

In the method of the present invention, steps (a) and (b) may be carried out continuously for 24 hours and the first time period may be 15 to 17 hours.

In the method of the present invention, an inorganic carbon source may be supplied in step (a).

In the method of the present invention, the organic carbon source may be selected from the group consisting of acetic acid, glucose, and mixtures thereof.

In the method of the present invention, the organic carbon source may be acetic acid at a concentration of 0.5 to 1.8 g/L.

In the method of the present invention, the organic carbon source may be glucose at a concentration of 0.15 to 0.7 g/L.

In the method of the present invention, the microalgal species may be *Chlorella protothecoides.*

The features and advantages of the present invention will become apparent from the following description with reference to the accompanying drawings.

Prior to the detailed description of the invention, it should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

The method of the present invention can maximize cell growth in large-scale microalgal cultivation by using the autotrophic mode in the light cycle and the heterotrophic mode in the dark cycle. The method of the present invention can achieve a high biomass yield without applying a load to the photosynthetic receptor system compared to existing autotrophic culture modes. The method of the present invention can achieve improved biomass productivity, which is the advantage of mixotrophic culture modes, and can avoid the problems of inhibited photosynthesis and poor conversion efficiency of organic carbon due to the supply of the organic carbon source during mixotrophic culture.

According to the method of the present invention, the concentration of the organic carbon source is maintained at a low level for the time period for the heterotrophic mode, avoiding the need to add additional chemicals such as antibiotics to prevent contamination with bacteria and other organisms. Therefore, the method of the present invention does not require any process for removing additional chemicals during biomass recovery, thus being advantageous from an economic viewpoint. In addition, acetic acid or glucose as the organic carbon source used in the dark cycle can be further supplied from various wastewaters, ensuring additional economic feasibility.

### BRIEF DESCRIPTION OF THE DRAWINGS

These and/or other aspects and advantages of the invention will become apparent and more readily appreciated from the following description of the embodiments, taken in conjunction with the accompanying drawings of which:
Fig. 1a is a schematic diagram showing a method for microalgal cultivation using a repeated sequential auto- and heterotrophic (RSAH) culture mode according to the present invention;
Fig. 1b shows curves comparing the trend of cell growth in a repeated sequential auto- and heterotrophy (RSAH) culture mode according to a method of the present invention with those in existing autotrophic and mixotrophic modes;
Figs. 2a to 2f show the experimental results of autotrophic, heterotrophic, and mixotrophic modes, where acetic acid as an organic carbon source and 5% CO₂ as an inorganic carbon source were supplied, in Example 1. 2a: Growth curves, 2b: specific growth rates measured at 24 h intervals, 2c: biomass production yields relative to the amounts of the organic carbon source consumed, 2d: ATP/ADP ratios measured at 24 h intervals, 2e: carbonic anhydrase specific activities measured at 48 h, and 2f: rubisco specific activities measured at 48 h;
Fig. 3a shows changes in dry cell weight when cultivated in an autotrophic mode and in a mixotrophic mode using different concentrations (0.5 g/L, 1 g/L, 2 g/L) of acetic acid as an organic carbon source in Example 2;
Fig. 3b shows the concentrations of intermediates (n mol (10⁷ cells)⁻¹) in the TCA cycle when cultivated in an autotrophic mode and in a mixotrophic mode using different concentrations (0.5 g/L, 1 g/L, 2 g/L) of acetic acid as an organic carbon source in Example 2;
Fig. 4a shows changes in dry cell weight when cultivated in an autotrophic mode and in a mixotrophic mode using different concentrations (0.5 g/L, 1 g/L, 2 g/L) of glucose as an organic carbon source in Example 3;
Fig. 4b shows the concentrations of intermediates (n mol (10⁷ cells)⁻¹) in the TCA cycle when cultivated in an autotrophic mode and in a mixotrophic mode using different concentrations (0.5 g/L, 1 g/L, 2 g/L) of glucose as an organic carbon source in Example 3;
Fig. 5a shows the dry cell weights of *Chlorella protothecoides* measured in different light/dark cycles (24 h/0 h, 16 h/8 h, 14 h/10 h, 12 h/12 h) in Example 4;
Fig. 5b shows changes in dry cell weight when cultivated in a repeated sequential auto- and heterotrophic culture mode using different concentrations (0.5 g/L, 1 g/L, 2 g/L) of acetic acid as an organic carbon source in Example 4;
Fig. 5c shows changes in dry cell weight when cultivated in a repeated sequential auto- and heterotrophic culture mode using different concentrations (0.167 g/L, 0.333 g/L, 0.667 g/L) of glucose as an organic carbon source in Example 4 (the time zones at the bottom in the graph indicate light cycles of 0-16 h, 24-40 h, 48-64 h, 72-88 h, and 96-112 h and dark cycles of 16-24 h, 40-48 h, 64-72 h, 88-96 h, and 112-120 h);
Fig. 6a shows changes in dry cell weight when cultivated in an autotrophic mode, a mixotrophic mode, and a repeated sequential auto- and heterotrophic culture mode using acetic acid as an organic carbon source in Example 5;
Fig. 6b shows changes in dry cell weight when cultivated in an autotrophic mode, a mixotrophic mode, and a repeated sequential auto- and heterotrophic culture mode using glucose as an organic carbon source in Example 5 (the time zones at the bottom in the graph indicate light cycles of 0-16 h, 24-40 h, 48-64 h, 72-88 h, and 96-112 h and dark cycles of 16-24 h, 40-48 h, 64-72 h, 88-96 h, and 112-120 h);
Fig. 6c shows specific growth rates relative to the amounts of acetic acid consumed as an organic carbon source for cultivation in a mixotrophic mode and a repeated sequential auto- and heterotrophic culture mode in Example 5;
Fig. 6d shows specific growth rates relative to the amounts of glucose consumed as an organic carbon source for cultivation in a mixotrophic mode and a repeated sequential auto- and heterotrophic culture mode in Example 5;
Fig. 6e shows ATP/ADP ratios when cultivated in an autotrophic mode, a mixotrophic mode, and a repeated sequential auto- and heterotrophic culture mode using acetic acid as an organic carbon source in Example 5; and
Fig. 6f shows ATP/ADP ratios when cultivated in an autotrophic mode, a mixotrophic mode, and a repeated sequential auto- and heterotrophic culture mode using glucose as an organic carbon source in Example 5.

### DETAILED DESCRIPTION OF THE INVENTION

The objects, specific advantages, and novel features of the present invention will become more apparent from the following detailed description and preferred embodiments, examples of which are illustrated in the accompanying drawings. Unless otherwise defined, all technical and scientific terms used herein have the same meaning as commonly understood by one of ordinary skill in the art to which this invention belongs. In general, the nomenclature used herein is well known and commonly employed in the art. In the description of the present invention, detailed explanations of related art are omitted when it is deemed that they may unnecessarily obscure the essence of the present invention.

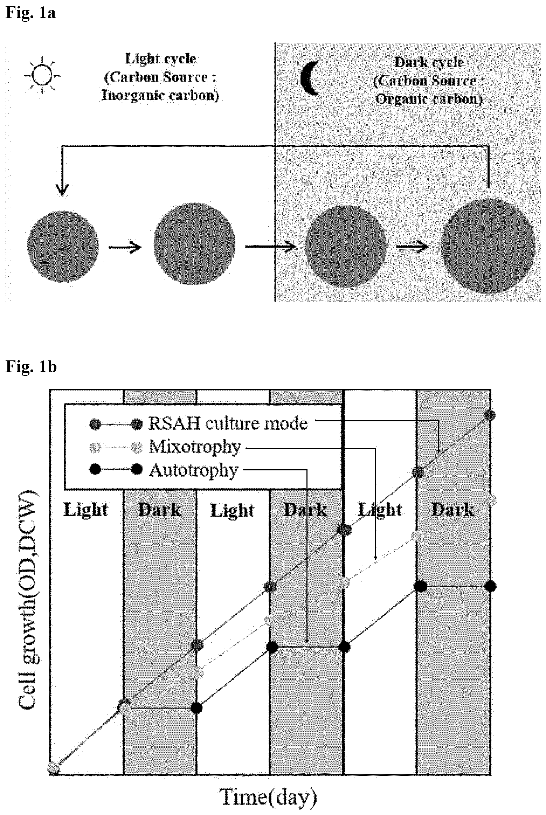

Fig. 1a is a schematic diagram showing a method for microalgal cultivation using a repeated sequential auto- and heterotrophic (RSAH) culture mode according to the present invention and Fig. 1b shows curves comparing the trend of cell growth in a repeated sequential auto- and heterotrophy (RSAH) culture mode according to a method of the present invention with those in existing autotrophic and mixotrophic modes.

As shown in Fig. 1a, the method of the present invention includes cultivating a microalgal species in a medium in an autotrophic mode for a predetermined first time period where light is supplied and supplying an organic carbon source to the medium to cultivate the microalgal species in a heterotrophic mode for a predetermined second time period where the supply of light is stopped.

Autotrophic, heterotrophic, and mixotrophic modes have been used to cultivate microalgae. In a broad sense, the present invention proposes a mixotrophic mode in which autotrophic and heterotrophic modes are sequentially used.

The method of the present invention employs an autotrophic mode and a heterotrophic mode that may proceed continuously. That is, the method of the present invention enables the cultivation of a microalgal species in an autotrophic mode for a first time period where light is supplied and in a heterotrophic mode for a second time period where the supply of light is stopped after the end of the first time period.

According to the method of the present invention, the autotrophic mode and the heterotrophic mode may be allowed to proceed continuously for 24 hours. In this case, the first time period may be 15 to 17 hours and the second time period may be 7 to 9 hours. For example, the microalgal species may be cultivated in the autotrophic mode during the daytime when photosynthesis occurs and in the heterotrophic mode at nighttime when photosynthesis does not occur.

According to the method of the present invention, the autotrophic mode and the heterotrophic mode may be repeated sequentially. That is, when the first time period during which light is supplied is defined as a light cycle and the second time period during which the supply of light is stopped is defined as a dark cycle, the microalgal species may be cultivated in the autotrophic mode in the light cycle and in the heterotrophic mode in the dark cycle while the light cycle and the dark cycle are repeated sequentially. Here, the light cycle and the dark cycle are divided into daytime and nighttime on a daily basis. The method of the present invention can be applied to outdoor microalgal cultivation but is not necessarily limited thereto as long as the light cycle is created by artificial light supply and the dark cycle is created by stopping the light supply. The light cycle and the dark cycle are preferably 16 hours and 8 hours, respectively.

A mixotrophic culture mode is advantageous in terms of biomass productivity over autotrophic and heterotrophic culture modes but the biomass productivity achieved by a mixotrophic culture mode is not always equal to the sum of those achieved by autotrophic and heterotrophic culture modes. Autotrophic and heterotrophic culture modes interact with each other depending on growth conditions during mixotrophic culture, resulting in the inhibition of cell growth in each mode. Typically, the addition of an organic carbon source affects the respiration of microalgae. Since an organic carbon source is more easily converted to an energy source than an inorganic carbon source, an increase in the concentration of the organic carbon source leads to a reduction in photosynthetic efficiency. The presence of an organic carbon source changes the activity of the TCA cycle to decrease photosynthetic efficiency. Acetates (acetic acid and sodium acetate) are mainly used as organic carbon sources for heterotrophic culture. Since an acetate tends to promote the production of succinic acid during assimilation, its addition at a high concentration inhibits cell growth. Further, the supply of an organic carbon source incurs an additional cost compared to the supply of CO₂ from flue gas. Furthermore, continuous light irradiation (for 16 hours or more) during autotrophic culture causes damage to the photosynthetic receptor system, leading to reduced photosynthetic efficiency and limited cell growth.

In an attempt to solve the problems of conventional mixotrophic culture modes, a periodic auto- and heterotrophic culture mode was designed in the present invention in which autotrophic and heterotrophic modes are allowed to proceed in different cycles to maximize the efficiency of each mode, and at the same time, an organic carbon source is added at a low concentration in the cycle for the heterotrophic mode to maximize cell growth.

The organic carbon source supplied during the heterotrophic culture in the method of the present invention may be selected from the group consisting of acetic acid, glucose, and mixtures thereof. The organic carbon source may be acetic acid at a concentration of 0.5 to 1.8 g/L, preferably 0.8 to 1.2 g/L, more preferably 1 g/L or glucose at a concentration of 0.15 to 0.7 g/L, preferably 0.5 to 0.7 g/L, more preferably 0.667 g/L. The organic carbon source should be consumed as completely as possible in the dark cycle to completely maintain the autotrophic mode in the light cycle. To this end, the concentration of the organic carbon source is set such that the largest possible amount of the organic carbon source is added as long as it does not interfere with cell growth.

A TAP-C medium may be used for the autotrophic mode and a TAP-C medium supplemented with glucose or acetic acid as the organic carbon source may be used for the heterotrophic mode.

CO₂ as an inorganic carbon source may be supplied during the autotrophic culture.

The method of the present invention can be used to cultivate *Chlorella protothecoides* but the target microalgal species is not particularly limited.

According to the method of the present invention, the autotrophic mode and the heterotrophic mode proceed separately. Since only the heterotrophic mode proceeds in the dark cycle where photosynthesis does not occur, an increase in biomass productivity can be expected without damage to the photosynthetic receptor system and the reduction of photosynthesis rate and the inhibition of cell growth due to the organic carbon source supplied in the mixotrophic mode can be prevented.

As shown in Fig. 1b, the repeated sequential auto- and heterotrophic (RSAH) culture mode employed in the method of the present invention ensures sufficient cell growth even in the dark cycle. Therefore, the RSAH culture mode enables the production of biomass in a higher yield than existing autotrophic and mixotrophic culture modes expect.

Overall, the method of the present invention uses a repeated sequential auto- and heterotrophic culture mode in which an autotrophic environment is applied in a cycle where light is supplied and a heterotrophic environment is applied in a cycle where an organic carbon source is supplied at a low concentration such that cell growth is not inhibited. The repeated sequential auto- and heterotrophic culture mode can bring about an increase in biomass productivity compared to general mixotrophic culture modes. In addition, the repeated sequential auto- and heterotrophic culture mode maintains the carbon fixation rate at a similar level to general autotrophic culture modes because it does not reduce photosynthesis. Furthermore, the repeated sequential auto- and heterotrophic culture mode is effective in preserving the carbon dioxide reduction efficiency.

The culture mode employed in the method of the present invention can maintain the concentration of the organic carbon source in the heterotrophic mode at a low level such that the conversion efficiency of the organic carbon source to biomass is maintained at a high level. Moreover, the method of the present invention eliminates the need to supply a high concentration of the organic carbon source. Therefore, the organic carbon source can be supplied from inexpensive wastewater, achieving economic feasibility.

The present invention will be more specifically explained with reference to the following examples.

### Example 1. Effects of autotrophic, heterotrophic, and mixotrophic modes on cell growth of Chlorella protothecoides

An experiment was conducted to determine the effects of autotrophic, heterotrophic, mixotrophic modes on the cell growth and metabolism of *Chlorella protothecoides.* To this end, acetic acid and CO₂ were used as an organic carbon source and an inorganic carbon source, respectively. The inorganic carbon source was used in an autotrophic mode, the organic carbon source was used in a heterotrophic mode, and both the organic carbon source and the inorganic carbon source were used in a mixotrophic mode to cultivate *Chlorella protothecoides.* Other physicochemical parameters, including temperature, pH, cultivation time, and inoculation amount, were maintained constant.

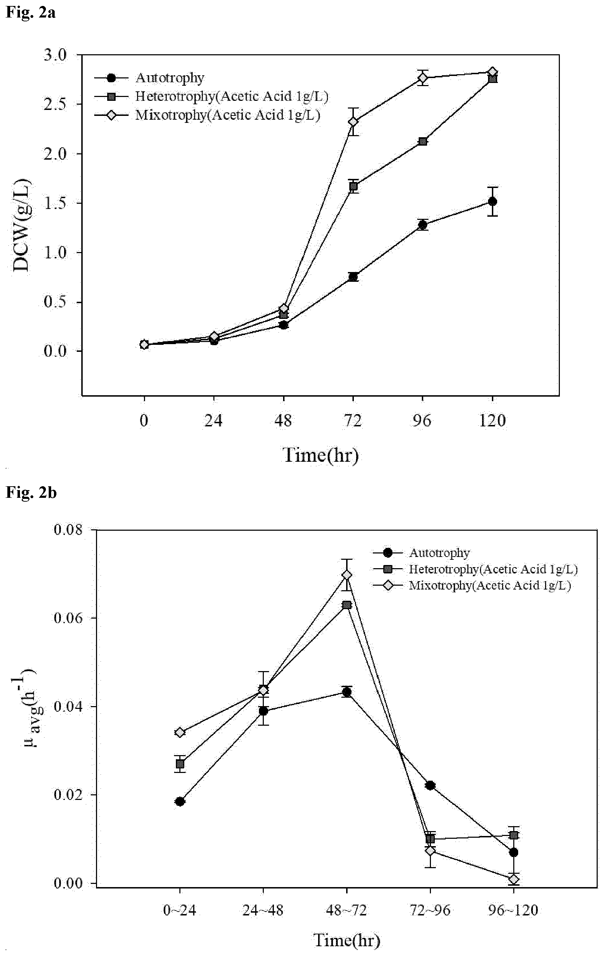

Figs. 2a to 2f show the experimental results of autotrophic, heterotrophic, and mixotrophic modes, where acetic acid as the organic carbon source and 5% CO₂ as the inorganic carbon source were supplied (2a: Growth curves, 2b: specific growth rates measured at 24 h intervals, 2c: biomass production yields relative to the amounts of the organic carbon source consumed, 2d: ATP/ADP ratios measured at 24 h intervals, 2e: carbonic anhydrase specific activities measured at 48 h, and 2f: rubisco specific activities measured at 48 h).

The experiment was continued under the above cultivation conditions for 120 h. As a result, the amount of biomass obtained in the mixotrophic mode at 72 h where cell growth was active was larger than that obtained in the autotrophic mode and that obtained in the heterotrophic mode but was smaller than the sum of the amounts of biomass obtained in the autotrophic and heterotrophic modes (see Fig. 2a).

The specific growth rate (µ) of the microalgal species in the mixotrophic mode at 48-72 h where cell growth was active was higher than that in the autotrophic mode and that in the heterotrophic mode but was lower than the sum of the specific growth rates in the autotrophic and heterotrophic modes (see Fig. 2b).

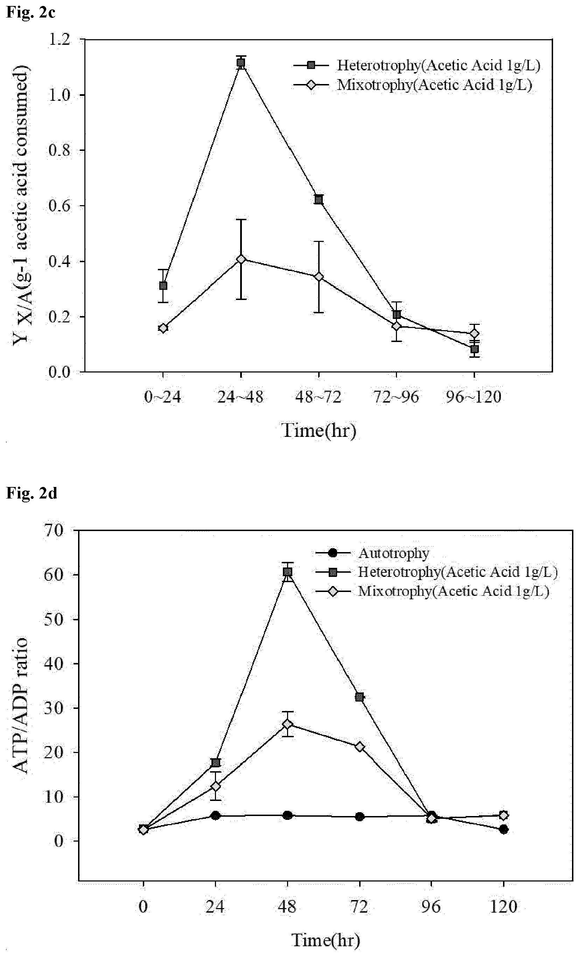

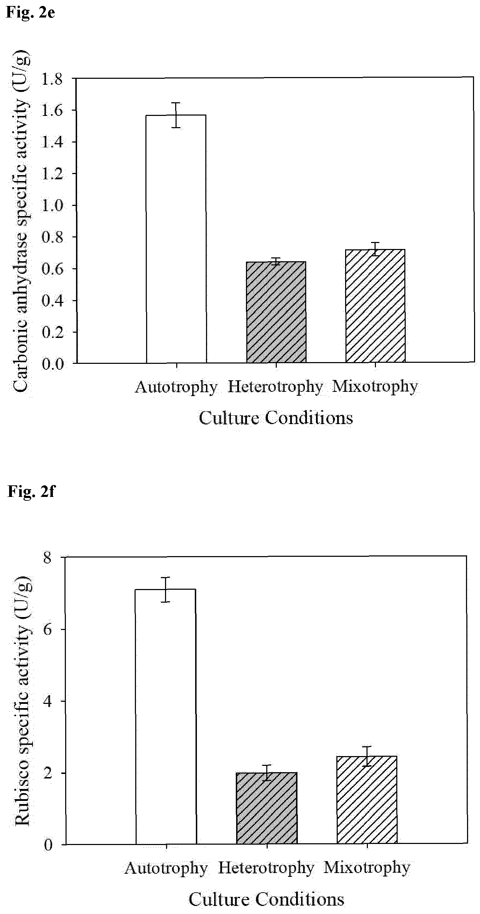

The biomass growth ratio (Y_{A/B}) relative to the amount of the organic carbon sources consumed in the heterotrophic mode was higher than that in the mixotrophic mode (see Fig. 2c). This result indicates that when the same amount of the organic carbon source was consumed, the conversion of the organic carbon source to biomass in the heterotrophic mode was higher than in the mixotrophic mode. To find the cause of the higher conversion in the heterotrophic mode, the ATP/ADP ratios in each cultivation environment were measured at 24 h intervals. The ATP/ADP ratio is indicative of how active the TCA cycle is. It can be said that the higher the ATP/ADP ratio, the higher the activity of the TCA cycle. As a result of the measurement, the ATP/ADP ratio in the heterotrophic mode was higher than that in the mixotrophic mode (see Fig. 2d). Referring to Fig. 2d together with Fig. 2c, the organic carbon source was efficiently used in the TCA cycle in the heterotrophic mode and the resulting conversion of the organic carbon source to biomass was higher than that in the mixotrophic mode. On the other hand, since the microalgal species can use the organic carbon source without photosynthesis, it prefers to use the organic carbon source compared to the inorganic carbon source in a state in which the inorganic carbon source and the organic carbon source coexist. That is, when the inorganic carbon source and the organic carbon source coexist, the photosynthetic efficiency of the microalgal species decreases, bringing about a reduction in the amount of carbon fixed from the inorganic carbon source. Carbonic anhydrase (CA) and rubisco are two key enzymes in the carbon dioxide concentrating mechanism (CCM). The degree of activation of carbon fixation can be measured depending on the activities of the two enzymes. The degrees of activity of carbonic anhydrase (see Fig. 2e) and rubisco (see Fig. 2f) in each culture mode (autotrophic, heterotrophic, and mixotrophic mode) were measured at 48 h where the TCA cycle was most active. The degree of activity of carbonic anhydrase in the autotrophic mode was ~2.67 times higher than those in the heterotrophic and mixotrophic modes. The degree of activity of carbonic anhydrase in the heterotrophic mode was similar to that in the mixotrophic mode. Likewise, the degree of activity of rubisco in the autotrophic mode was ~3.5 times higher than those in the heterotrophic and mixotrophic modes. The degree of activity of rubisco in the heterotrophic mode was similar to that in the mixotrophic mode. In conclusion, the carbon fixation efficiency of the inorganic carbon source through the carbon dioxide concentrating mechanism in the mixotrophic mode is lower than that in the autotrophic mode. In addition, considering the carbon dioxide concentrating mechanism and the efficient conversion of the organic carbon source to biomass, the cultivation in the separate autotrophic and heterotrophic modes can be said to be more efficient than that in the mixotrophic mode in the presence of the same amount of the carbon source.

### Example 2. Comparison of biomass and TCA cycle intermediates in mixotrophic mode using acetic acid as organic carbon source and in autotrophic mode using inorganic carbon source

Biomass and TCA cycle intermediates in the mixotrophic mode were compared with those in the autotrophic mode when acetic acid was supplied as an organic carbon source. For photosynthesis, light was continuously supplied during the growth period in the mixotrophic and autotrophic modes. In this experiment, CO₂ and acetic acid were used as inorganic and organic carbon sources, respectively, and their concentrations were changed to 0.5 g/L, 1 g/L, and 2 g/L. A flask in the autotrophic culture mode was used as a control of the mixotrophic mode and CO₂ was used as an inorganic carbon source. Light was continuously supplied in the heterotrophic and autotrophic modes, as in a general cultivation mode at a laboratory scale.

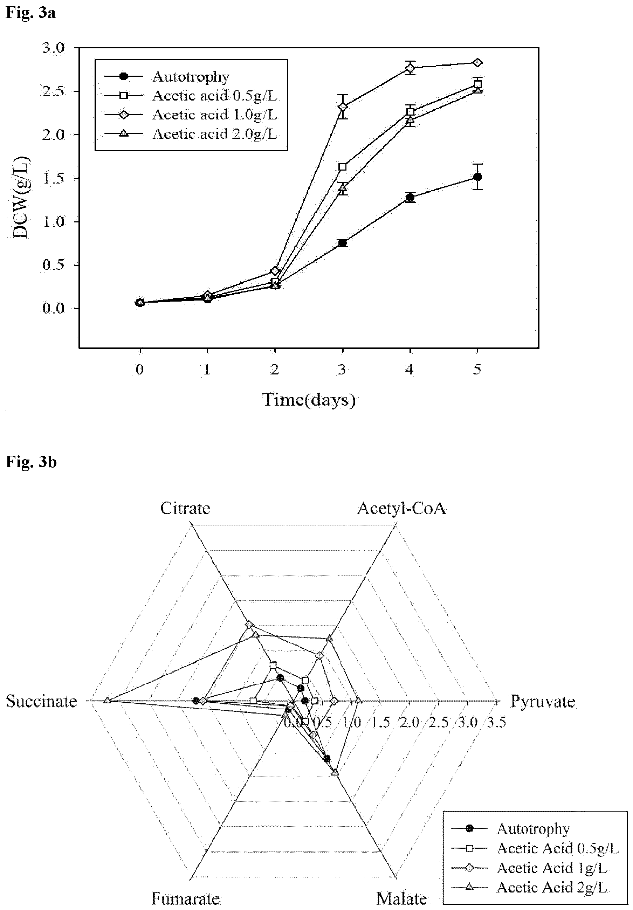

Fig. 3a shows changes in dry cell weight when cultivated in the autotrophic mode and in the mixotrophic mode using different concentrations (0.5 g/L, 1 g/L, 2 g/L) of acetic acid as the organic carbon source. Fig. 3b shows the concentrations of intermediates (*n* mol (10⁷ cells)⁻¹) in the TCA cycle when cultivated in the autotrophic mode and in the mixotrophic mode using different concentrations (0.5 g/L, 1 g/L, 2 g/L) of acetic acid as the organic carbon source.

Fig. 3a shows the amounts of biomass obtained by all the proposed culture modes and Fig. 3b and Table 1 show the amounts of TCA cycle intermediates measured at 60 h where cell growth was most active. In general, as the amount of an organic carbon source supplied increases, the amount of resulting biomass tends to increase. However, the use of acetic acid as the organic carbon source showed large deviations from this general tendency. When the largest amount of the carbon source (2 g/L acetic acid) was supplied, the smallest dry cell weight (DCW) was obtained at 72-120 h. The supply of 1 g/L acetic acid as the organic carbon source led to the largest dry cell weight (DCW), which was ~12% larger than the supply of 2 g/L acetic acid (see Fig. 3a). These results concluded that the initial concentration of acetic acid is not proportional to cell growth even when other conditions such as pH and light intensity are the same.

The inhibition of cell growth was analyzed when high concentrations of acetic acid were used. To this end, intermediates of the TCA cycle at the concentrations of acetic acid were analyzed using a UPLC system. In the TCA cycle, the organic carbon source is converted to pyruvate, acetyl-CoA, citrate, succinate, fumarate, and malate in this order. However, the use of acetic acid as an organic carbon source for the growth of microalgae leads to excessive production of succinate that is known to inhibit cell growth. Referring to Fig. 3b and Table 1, the supply of 2 g/L acetic acid as an organic carbon source led to the production of a larger amount of succinate as an intermediate than the supply of a lower concentration of acetic acid in the autotrophic mode. In addition, the use of a high concentration of acetic acid increases the effect of inhibiting ATP and inhibits the activation of the TCA cycle to interfere with cell growth. In conclusion, the supply of an organic carbon source above a predetermined concentration can interfere with rapid cell growth for large-scale cultivation.

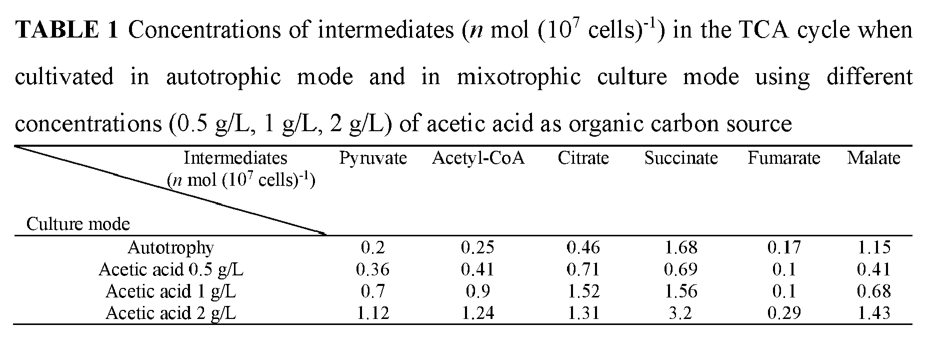

### Example 3. Comparison of biomass and TCA cycle intermediates in mixotrophic mode using glucose as organic carbon source and in autotrophic mode using inorganic carbon source

Biomass and TCA cycle intermediates in the autotrophic mode were compared with those in the mixotrophic mode when glucose was supplied as an organic carbon source. For photosynthesis, light was continuously supplied during the growth period in the mixotrophic and autotrophic modes. CO₂ and different concentrations (0.167 g/L, 0.333 g/L, and 0.667 g/L) of glucose were used as inorganic and organic carbon sources, respectively. The amount of carbon in 0.167 g/L glucose is the same as that in 0.5 g/L acetic acid. The amounts of carbon in 0.333 g/L and 0.667 g/L glucose are the same as those in 1 g/L and 2 g/L acetic acid, respectively.

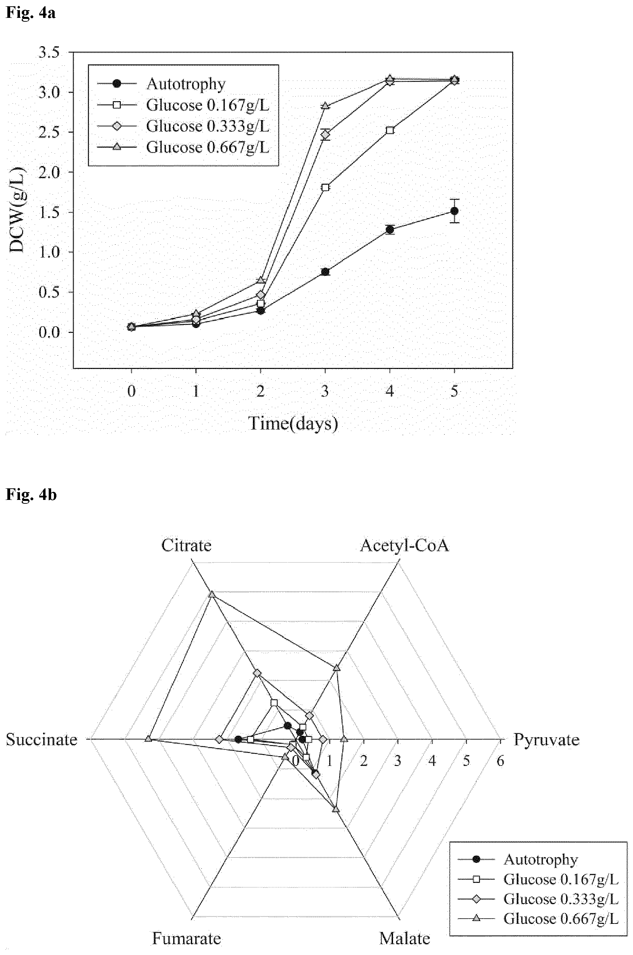

Fig. 4a shows changes in dry cell weight when cultivated in the autotrophic mode and in the mixotrophic mode using different concentrations (0.5 g/L, 1 g/L, 2 g/L) of glucose as the organic carbon source. Fig. 4b shows the concentrations of intermediates *(n* mol (10⁷ cells)⁻¹) in the TCA cycle when cultivated in the autotrophic mode and in the mixotrophic mode using different concentrations (0.5 g/L, 1 g/L, 2 g/L) of glucose as the organic carbon source.

The use of glucose as the organic carbon source showed a similar tendency to that of the use of a low concentration of acetic acid as the organic carbon source but showed a different tendency as the concentration of acetic acid as the organic carbon source increased. The amounts obtained in all culture modes are shown in Fig. 4a. After 4-day culture, the growth rate in the mixotrophic mode increased with increasing amount of glucose as the organic carbon source, which was different from when acetic acid was added but coincided with the general tendency. However, the amount of biomass did not increase in direct proportion to the amount of the organic carbon source added.

Intermediates of the TCA cycle measured at 60 h where cell growth was most active were analyzed and are shown in Fig. 4b and Table 2. Citrate was obtained at a higher concentration when 0.667 g/L glucose was supplied than when 0.333 g/L glucose was supplied. Citrate slows down cell growth. In addition, excess acetyl-CoA and citrate cause gluconeogenesis to inhibit glycolysis. The inhibition of glycolysis leads to a decreases in pyruvate and acetyl-CoA which play important roles in cell growth. As a result, the production yield of biomass was found to decrease with increasing amount of the organic carbon source.

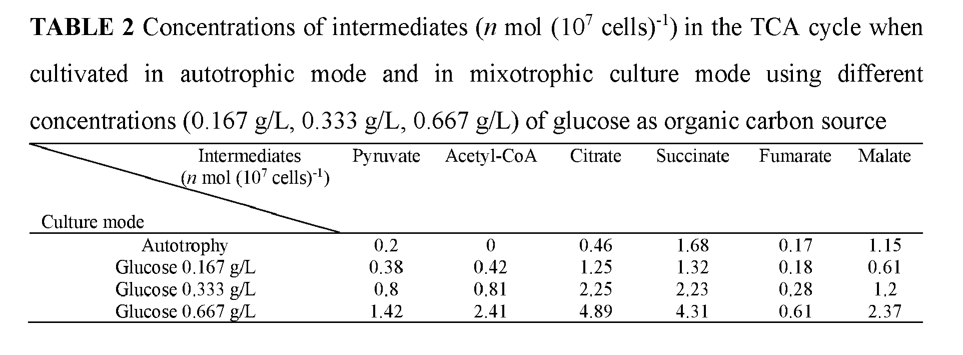

### Example 4. Repeated sequential auto- and heterotrophic culture mode using acetic acid or glucose

The results of Examples 2 and 3 demonstrated that the supply of an appropriate amount of the organic carbon source can achieve high cell growth relative to the amount of the organic carbon source consumed. Long-term exposure to light may cause damage to the photosynthetic receptor system, leading to inhibition of photosynthesis.

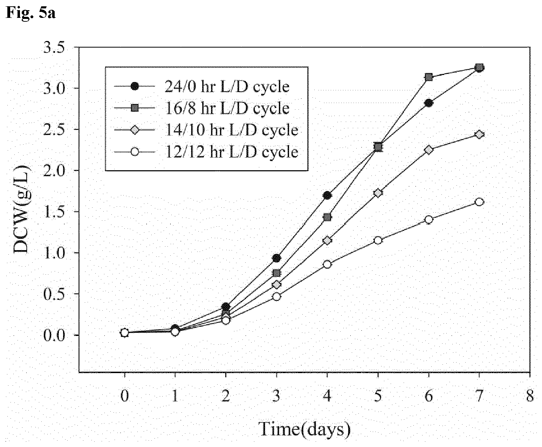

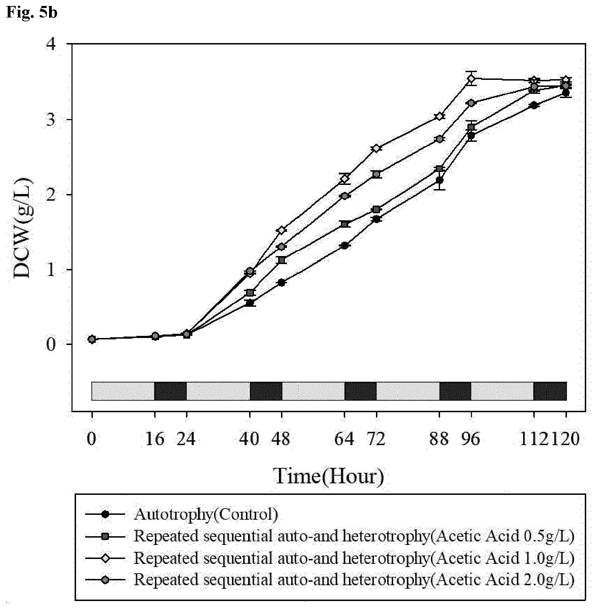

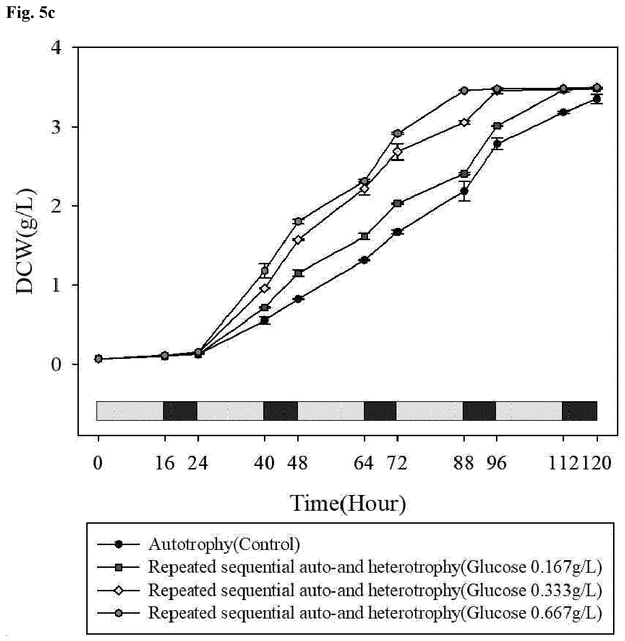

Fig. 5a shows the dry cell weights of *Chlorella protothecoides* measured in different light/dark cycles (24 h/0 h, 16 h/8 h, 14 h/10 h, 12 h/12 h), Fig. 5b shows changes in dry cell weight when cultivated in the repeated sequential auto- and heterotrophic culture mode using different concentrations (0.5 g/L, 1 g/L, 2 g/L) of acetic acid as the organic carbon source, and Fig. 5c shows changes in dry cell weight when cultivated in the repeated sequential auto- and heterotrophic culture mode using different concentrations (0.167 g/L, 0.333 g/L, 0.667 g/L) of glucose as the organic carbon source. The time zones at the bottom in the graph indicate light cycles of 0-16 h, 24-40 h, 48-64 h, 72-88 h, and 96-112 h and dark cycles of 16-24 h, 40-48 h, 64-72 h, 88-96 h, and 112-120 h.

The highest growth rate of *Chlorella protothecoides* was achieved when the light cycle/dark cycle was 16 h/8 h (see Fig. 5a). In addition, existing mixotrophic modes are disadvantageous in that a high concentration of a substrate inhibits photosynthesis, resulting in low microalgal productivity and poor carbon fixation efficiency. In an attempt to overcome these disadvantageous (reduced carbon fixation efficiency and inhibited photosynthesis), the present invention proposes a repeated sequential auto- and heterotrophic culture mode in which an autotrophic mode is used in the light cycle and a heterotrophic mode is used in the dark cycle with supply of a low concentration of an organic carbon source. The use of the proposed mode can overcome the disadvantages of mixotrophic culture modes and enables the use of an additional organic carbon source for large-scale cultivation using concentrated carbon dioxide to achieve improved biomass productivity and shorten the cell culture cycle.

An experiment was conducted using acetic acid (0.5 g/L, 1 g/L, 2 g/L) or glucose (0.167 g/L, 0.333 g/L, 0.667 g/L) as an organic carbon source in the repeated sequential auto- and heterotrophic culture mode. The results are compared in Figs. 5b and 5c. The light cycle/dark cycle was set to 16 h/8 h. Light and 5% CO₂ were supplied at 0-16 h, 24-40 h, 48-64 h, 72-88 h, and 96-112 h corresponding to the light cycles. A predetermined concentration of the organic carbon source as a substrate was supplied at 16-24 h, 40-48 h, 64-72 h, 88-96 h, and 112-120 h corresponding to the dark cycles. An autotrophic mode in which light and 5% CO₂ were continuously supplied was used as a control.

When acetic acid was used as a substrate, continuous supply of 1 g/L acetic acid in the dark cycle led to the highest growth rate and improved biomass growth by 27.2% at 96 h compared to the control (no additional biomass growth occurred after 96 h due to nitrogen source depletion). The continuous supply of 2 g/L acetic acid in the dark cycle led to the formation of succinate to inhibit cell growth, and as a result, it slowed down biomass growth compared to the continuous supply of 1 g/L acetic acid (see Fig. 5b).

When glucose was used as a substrate, continuous supply of the highest concentration (0.667 g/L) of glucose as a substrate in the dark cycle led to high biomass growth and improved biomass growth by 58.1% at 88 h compared to the control (no additional biomass growth occurred after 88 h due to nitrogen source depletion).

In conclusion, the repeated sequential auto- and heterotrophic culture mode is more effective than existing autotrophic culture modes.

### Example 5. Repeated sequential auto- and heterotrophic culture mode for maximizing conversion to biomass when acetic acid and glucose were used as organic carbon sources

When the same amount of a substrate is added to cultivate a microalgal species in the repeated sequential auto- and heterotrophic culture mode, the amount of the substrate per microalgal cell decreases and the cell growth rate decreases with increasing cultivation time. Thus, it is necessary to increase the amount of the substrate added in the dark cycle in proportion to the amount of microalgal cells in order to keep the cell growth rate. An autotrophic mode in which the light cycle was maintained for 24 h with supply of 5% CO₂ was used as a control. Acetic acid as an organic carbon source was added at concentrations of 0.5 g/L, 1 g/L, 1.5 g/L, and 1.75 g/L in dark cycles of 16-24 h, 40-48 h, 64-72 h, and 88-96 h, respectively, in the repeated sequential auto- and heterotrophic culture mode and the organic carbon source was added in an amount (4.75 g/L) such that the amount of the substrate was the same in the mixotrophic mode. Thereafter, dry weights were measured. When glucose as an organic carbon source was added at concentrations of 0.167 g/L, 0.333 g/L, 0.5 g/L, 0.583 g/L in dark cycles of 16-24 h, 40-48 h, 64-72 h, and 88-96 h, respectively, in the repeated sequential auto- and heterotrophic culture mode and glucose was added in an amount (1.583 g/L) such that the amount of the substrate was the same in the mixotrophic mode. Thereafter, dry weights were measured.

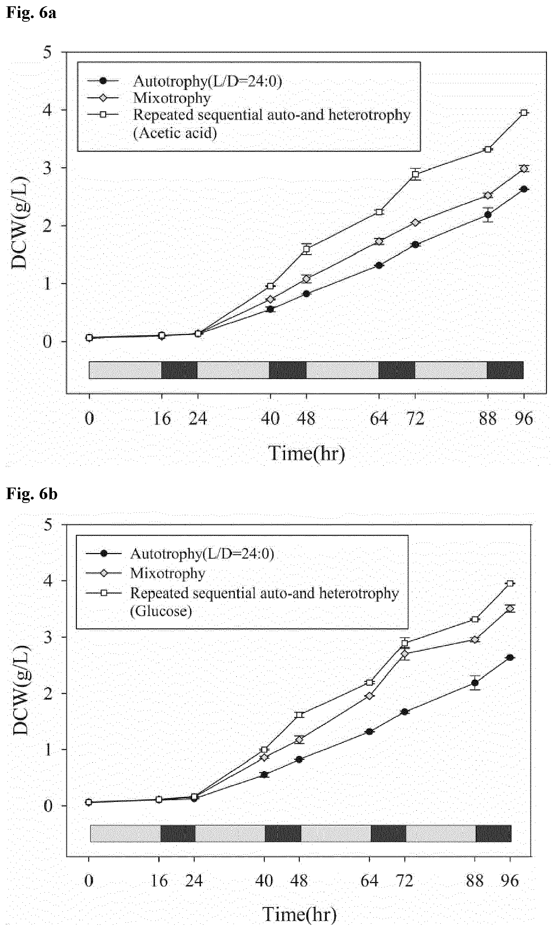

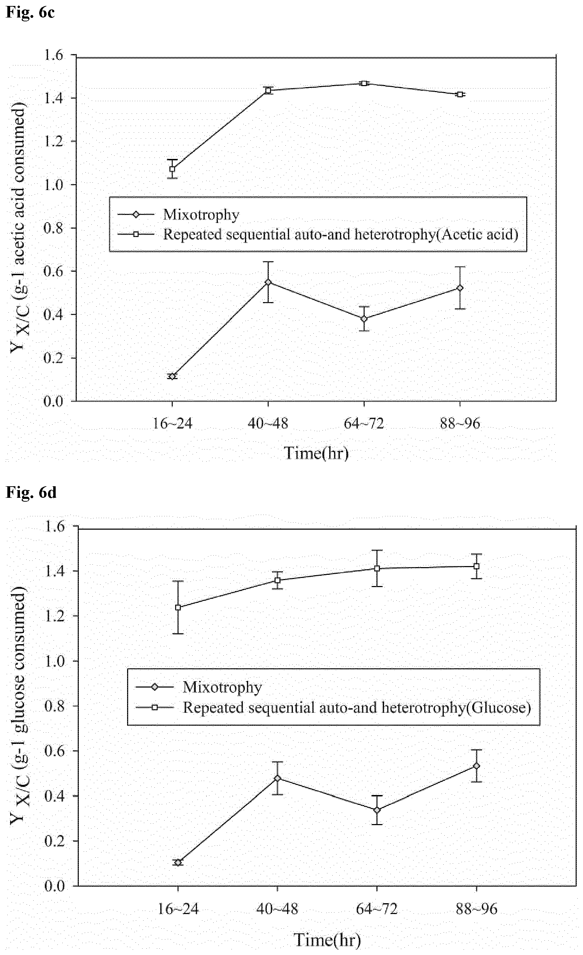

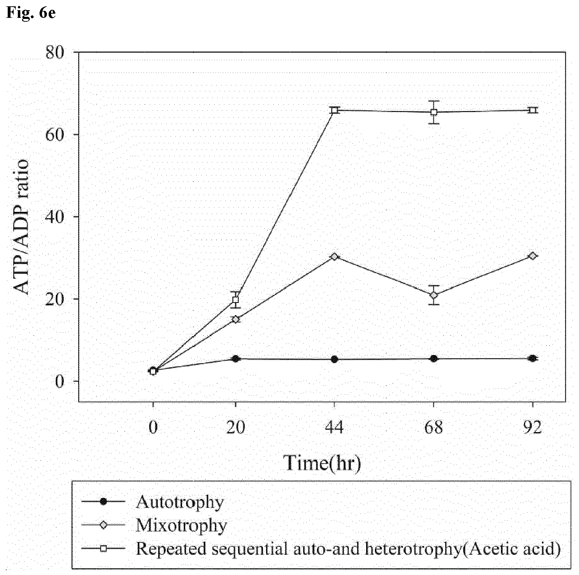

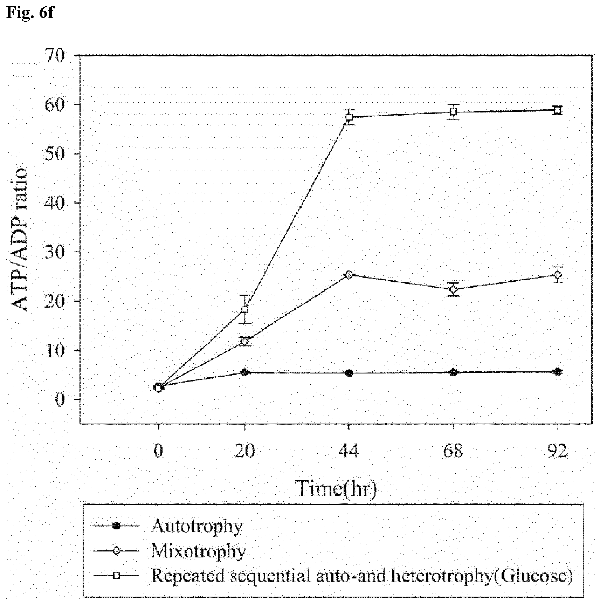

Fig. 6a shows changes in dry cell weight when cultivated in the autotrophic mode, the mixotrophic mode, and the repeated sequential auto- and heterotrophic culture mode using acetic acid as the organic carbon source. Fig. 6b shows changes in dry cell weight when cultivated in the autotrophic mode, the mixotrophic mode, and the repeated sequential auto- and heterotrophic culture mode using glucose as the organic carbon source. The time zones at the bottom in the graph indicate light cycles of 0-16 h, 24-40 h, 48-64 h, 72-88 h, and 96-112 h and dark cycles of 16-24 h, 40-48 h, 64-72 h, 88-96 h, and 112-120 h. Fig. 6c shows specific growth rates relative to the amounts of acetic acid consumed as the organic carbon source for cultivation in the mixotrophic mode and the repeated sequential auto- and heterotrophic culture mode. Fig. 6d shows specific growth rates relative to the amounts of glucose consumed as the organic carbon source for cultivation in the mixotrophic mode and the repeated sequential auto- and heterotrophic culture mode. Fig. 6e shows ATP/ADP ratios when cultivated in the autotrophic mode, the mixotrophic mode, and the repeated sequential auto- and heterotrophic culture mode using acetic acid as the organic carbon source. Fig. 6f shows ATP/ADP ratios when cultivated in the autotrophic mode, the mixotrophic mode, and the repeated sequential auto- and heterotrophic culture mode using glucose as the organic carbon source.

The cell growth rate at 96 h in the repeated sequential auto- and heterotrophic culture mode using acetic acid as the substrate was 32.3% higher than that in the mixotrophic mode and 50.2% higher than that in the autotrophic mode (see Fig. 6a). Since the organic carbon source was added in proportion to the amount of microalgal cells in the repeated sequential auto- and heterotrophic culture mode, the yield (Y_{X/A}) relative to the amount of the substrate consumed in the repeated sequential auto- and heterotrophic culture mode was maintained at a similar level at 40-96 h and was ≥ 2.59 times higher than that in the mixotrophic mode (see Fig. 6c). The ATP/ADP ratio in the repeated sequential auto- and heterotrophic culture mode was ≥ 2.17 times higher than that in the mixotrophic mode (see Fig. 6e). The ATP/ADP ratio is indicative of the activation of the TCA cycle.

The cell growth rate at 96 h in the repeated sequential auto- and heterotrophic culture mode using glucose as the substrate was 12.6% higher than that in the mixotrophic mode and 50.1% higher than that in the autotrophic mode (see Fig. 6b). The addition of the organic carbon source in proportion to the amount of microalgal cells maintained the activation of the TCA cycle at a high level, leading to a higher yield (Y_{X/A}) relative to the amount of the substrate consumed (see Figs. 6d and 6f).

The carbonic anhydrase specific activity and rubisco specific activity in the repeated sequential auto- and heterotrophic culture mode were also 84.8% and 149% higher than those in the mixotrophic mode, respectively. The enzyme specific activities are indicative of the carbon concentrating mechanism (CCM). As shown in Table 3, the maximum carbon fixation rate in the repeated sequential auto- and heterotrophic culture mode was 42.3% higher than that in the autotrophic mode due to the higher productivity in the repeated sequential auto- and heterotrophic culture mode.

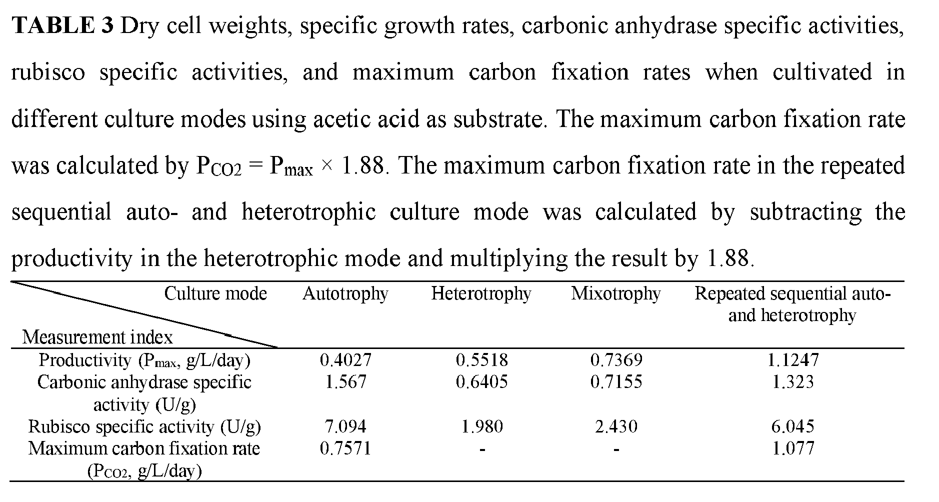

In conclusion, the repeated sequential auto- and heterotrophic culture mode promotes the activation of the TCA cycle while avoiding a reduction in photosynthetic efficiency, contributing to cell growth. In addition, since the preservation of photosynthetic efficiency has a great influence on carbon fixation, the repeated sequential auto- and heterotrophic culture mode can also be considered a significant culture mode from the point of view of CCU.

Although the present invention has been described herein with reference to the specific embodiments, these embodiments do not serve to limit the invention and are set forth for illustrative purposes. It will be apparent to those skilled in the art that modifications and improvements can be made without departing from the spirit and scope of the invention.

Such simple modifications and improvements of the present invention belong to the scope of the present invention, and the specific scope of the present invention will be clearly defined by the appended claims.

## Claims

1. A method for microalgal cultivation comprising (a) cultivating a microalgal species in a medium in an autotrophic mode for a predetermined first time period where light is supplied and (b) supplying an organic carbon source to the medium to cultivate the microalgal species in a heterotrophic mode for a predetermined second time period where the supply of light is stopped.

2. The method according to claim 1, wherein steps (a) and (b) are repeated sequentially.

3. The method according to claim 1, wherein steps (a) and (b) are carried out continuously for 24 hours and the first time period is 15 to 17 hours.

4. The method according to claim 1, wherein an inorganic carbon source is supplied in step (a).

5. The method according to claim 1, wherein the organic carbon source is selected from the group consisting of acetic acid, glucose, and mixtures thereof.

6. The method according to claim 5, wherein the organic carbon source is acetic acid at a concentration of 0.5 to 1.8 g/L.

7. The method according to claim 5, wherein the organic carbon source is glucose at a concentration of 0.15 to 0.7 g/L.

8. The method according to claim 1, wherein the microalgal species is *Chlorella protothecoides.*
